# EUROPÄISCHE PATENTSCHRIFT

(11) **EP 2 664 309 B1**
(45) Veröffentlichungstag und Bekanntmachung des Hinweises auf die Patenterteilung: **29.06.2016**
(21) Anmeldenummer: 12003793.2
(22) Anmeldetag: 14.05.2012
(51) Int. Cl.: A61F 9/008

(54) **Vorrichtung zur Behandlung von Augengewebe**
Device for treating eye tissue
Dispositif de traitement de tissu oculaire

(43) Veröffentlichungstag der Anmeldung: 20.11.2013
(73) Patentinhaber: Ziemer Ophthalmic Systems AG, 2562 Port (CH)
(72) Erfinder: Rathjen, Christian, 28197 Bremen (DE)
(74) Vertreter: Rentsch Partner AG

(56) Entgegenhaltungen:
- EP-A1- 1 731 120
- WO-A2-2010/075571

## Beschreibung

### Technisches Gebiet

Die vorliegende Erfindung betrifft eine ophthalmologische Vorrichtung zur Behandlung von Augengewebe mittels Laserpulsen. Die vorliegende Erfindung betrifft insbesondere eine ophthalmologische Vorrichtung zur Behandlung von Augengewebe, welche eine Lichtquelle zur Erzeugung der Laserpulse und einen Lichtprojektor zur fokussierten Projektion der Laserpulse ins Augengewebe umfasst.

### Stand der Technik

Fehlsichtigkeiten wie Myopie (Kurzsichtigkeit), Hyperopie (Weitsichtigkeit oder Übersichtigkeit) oder Astigmatismus (Stabsichtigkeit) können heute durch refraktiv-chirurgische Behandlung dauerhaft korrigiert werden. Refraktiv-chirurgische Behandlungen sind chirurgische Eingriffe am Auge, die die optische Brechkraft des Auges ändern mit dem Ziel, diese einem gewünschten Wert möglichst gut anzunähern. Eines der bedeutendsten Verfahren in der refraktiven Chirurgie ist die so genannte Laser in-situ Keratomileusis (LASIK), bei der das Innere der Hornhaut mit einem Excimerlaser abgetragen wird, nachdem zuvor ein Hornhautlappen teilweise abgetrennt und weggeklappt wird. Solche Hornhautlappen werden mit mechanischen Mikrokeratomen oder mittels stark fokussierter Femtosekundenlaserpulsen geschnitten. Geeignete Femtosekundenlasersysteme erzeugen Laserpulse mit Pulsbreiten von typisch 100fs bis 1000fs (1fs=10-15s).

In EP 1 731 120 wird ein System zum Schneiden eines Hornhautlappens mittels Femtosekundenlaserpulsen beschrieben, das eine Basisstation umfasst, in welcher die Lichtquelle zur Erzeugung der Femtolaserpulse angeordnet ist. Die Vorrichtung umfasst einen Lichtprojektor, welcher in einem Applikationskopf angeordnet ist, der über einen Spiegelgelenkarm flexibel an der Basisstation angebracht ist und eine manuelle Applikation des Applikationskopfs und Lichtprojektors auf das Auge eines Patienten ermöglicht. Um das Gewicht des Applikationskopfs für die manuelle Applikation mittels des Spiegelgelenkarms zu ermöglichen weist der Lichtprojektor gegenüber den vorgenannten Systemen kleiner und damit leichter dimensionierte Linsensysteme auf. Um trotz des kleiner dimensionierten Linsensystems zum Schneiden des Gewebelappens auf dem Auge ein ausgedehntes Bearbeitungsgebiet mit fokussierten Laserpulsen bearbeiten zu können, verfügt der Applikationskopf zudem über Bewegungstreiber zum Verfahren des Lichtprojektors in mehreren Bearbeitungsrichtungen. Beim Verfahren des Lichtprojektors wird dessen Position hinsichtlich der Lichtquelle verändert, so dass die Länge des Lichtübertragungspfads von der Lichtquelle zum Lichtprojektor verändert wird.

WO 2010/075571 beschreibt ein Lasersystem für die Augenchirurgie, in welchem ein OCT System in den Strahlengang des Lasersystems eingekoppelt ist. Aufgrund eines beweglichen Fokussierobjektivs kann sich die optische Pfadlänge des OCT Systems verändern. Gemäss WO 2010/075571 wird die Objektivposition erfasst und in einem Algorithmus zur entsprechenden Kompensation der OCT Daten verwendet. In einer alternativen Variante wird ein Spiegel des Referenzarms des OCT Systems fest mit dem beweglichen Objektiv verbunden ist, so dass sich die Pfadlänge automatisch mit der Bewegung des Objektivs anpasst.

### Darstellung der Erfindung

Es ist eine Aufgabe der vorliegenden Erfindung eine ophthalmologische Vorrichtung zur Behandlung von Augengewebe mittels Laserpulsen vorzuschlagen, welche zumindest einige Nachteile bekannter Vorrichtungen nicht aufweist. Es ist insbesondere eine Aufgabe der vorliegenden Erfindung eine ophthalmologische Vorrichtung zur Behandlung von Augengewebe mittels Laserpulsen vorzuschlagen, welche eine Erleichterung der Behandlung ermöglicht.

Gemäss der vorliegenden Erfindung werden diese Ziele durch die Merkmale der unabhängigen Ansprüche erreicht. Weitere vorteilhafte Ausführungsformen gehen ausserdem aus den abhängigen Ansprüchen und der Beschreibung hervor.

Eine ophthalmologische Vorrichtung zur Behandlung von Augengewebe mittels Laserpulsen umfasst eine Lichtquelle zur Erzeugung der Laserpulse und einen Lichtprojektor zur fokussierten Projektion der Laserpulse ins Augengewebe, wobei der Lichtprojektor bezüglich der genannten Lichtquelle bewegbar ist, so dass die Länge des Lichtübertragungspfads von der Lichtquelle zum Lichtprojektor veränderbar ist.

Die oben genannten Ziele werden durch die vorliegende Erfindung insbesondere dadurch erreicht, dass die ophthalmologische Vorrichtung überdies ein interferometrisches Messsystem zum Vermessen von Augenstrukturen umfasst. Das interferometrische Messsystem ermöglicht ein flexibles Vermessen der Augenstrukturen, ohne dass dazu die ophthalmologische Vorrichtung zur Behandlung des Augengewebe vom Patienten wegbewegt und stattdessen eine Messvorrichtung appliziert werden muss. Die dadurch ermöglichte flexible Vermessung der Augenstrukturen erleichtert die Behandlung indem aktuelle Messdaten der Augenstrukturen vor der Behandlung, während der Behandlung und nach der Behandlung einfach und effizient erfasst und bereitgestellt werden können.

In einer Ausführungsvariante ist das interferometrische Messsystem in den genannten Lichtübertragungspfad eingekoppelt. Die Einkopplung des interferometrischen Messsystems in den Lichtübertragungspfad von der Lichtquelle zum Lichtprojektor ermöglicht eine Vermessung der Augestrukturen an und aus der selben Position, aus und an der auch die Laserpulse zur Behandlung des Augengewebes projiziert werden.

Vorzugsweise umfasst die ophthalmologische Vorrichtung ein bezüglich der genannten Lichtquelle bewegbares optisches Element, das vorgesehen ist, eine Veränderung der Weglängendifferenz von Messarm und Referenzarm des interferometrischen Messsystems aufgrund einer durch eine Bewegung des Lichtprojektors bewirkten Veränderung der Länge des Lichtübertragungspfads zu verhindern. Dadurch können im interferometrischen Messsystem Messfehler aufgrund von Längenveränderungen des Lichtübertragungspfads vermieden werden, die durch Bewegungen des Lichtprojektors bezüglich der Lichtquelle verursacht werden.

Das bewegbare optische Element ist ein im Referenzarm angeordneter Spiegel und die ophthalmologische Vorrichtung umfasst ein Steuermodul und einen Bewegungstreiber, die eingerichtet sind, den Spiegel so zu verschieben, dass die Weglänge des Referenzarms entsprechend einer durch eine Bewegung des Lichtprojektors bewirkten Veränderung der Länge des Lichtübertragungspfads angepasst wird.

In einem Bespiel ist das bewegbare optische Element eine fest mit dem Lichtprojektor verbundene Lichtleiterkopplung, welche den Lichtprojektor über eine flexible Lichtfaser in das interferometrischen Messsystem einbindet.

In einem Beispiel ist das bewegbare optische Element ein fest mit dem Lichtprojektor verbundener Strahlteiler, über welchen der Messarm und der Referenzarm des interferometrischen Messsystems gekoppelt sind.

In einem Beispiel umfasst die ophthalmologische Vorrichtung einen mit dem Lichtprojektor fest verbundenen weiteren Lichtprojektor, welcher über einen flexiblen Lichtleiter in das interferometrische Messsystem eingebunden ist.

In einer Ausführungsvariante umfasst die ophthalmologische Vorrichtung einen auf das Auge applizierbaren Applikationskopf und der Lichtprojektor ist im Applikationskopf angeordnet. In einer Variante ist der Referenzarm des interferometrischen Messsystems im Applikationskopf angeordnet. In einer Variante umfasst das interferometrische Messsystem eine Lichtquelle, welche im Applikationskopf angeordnet ist. In einer Variante umfasst das interferometrische Messsystem einen Detektor, welcher im Applikationskopf angeordnet ist. In einer alternativen Variante umfasst das interferometrische Messsystem einen Detektor, welcher extern zum Applikationskopf angeordnet ist.

Die ophthalmologische Vorrichtung umfasst eine Basisstation, die Lichtquelle zur Erzeugung der Laserpulse ist fest in der Basisstation angeordnet, der Lichtprojektor ist über einen Arm mit der Basisstation verbunden, und der Referenzarm des interferometrischen Messsystems ist in der Basisstation angeordnet. In einer Variante umfasst das interferometrische Messsystem eine Lichtquelle, welche in der Basisstation angeordnet ist. In einer Variante umfasst das interferometrische Messsystem einen Detektor, welcher in der Basisstation angeordnet ist. In einer Ausführungsvariante weist das interferometrische Messsystem einen Referenzarm mit veränderbarer Referenzarmlänge auf.

### Kurze Beschreibung der Zeichnungen

Nachfolgend wird eine Ausführung der vorliegenden Erfindung anhand eines Beispieles beschrieben. Das Beispiel der Ausführung wird durch die folgenden beigelegten Figuren illustriert:
- Figur 1:: zeigt schematisch im Querschnitt eine ophthalmologische Vorrichtung zur Behandlung von Augengewebe mittels Laserpulsen, welche ein interferometrisches Messsystem zum Vermessen von Augenstrukturen aufweist, das in einem beweglichen Applikationskopf angeordnet ist.
- Figur 2:: zeigt schematisch im Querschnitt eine ophthalmologische Vorrichtung zur Behandlung von Augengewebe mittels Laserpulsen, welche ein interferometrisches Messsystem zum Vermessen von Augenstrukturen aufweist, das fest mit einem beweglichen Lichtprojektor verbunden ist.
- Figur 3:: zeigt schematisch im Querschnitt eine ophthalmologische Vorrichtung zur Behandlung von Augengewebe mittels Laserpulsen, welche ein interferometrisches Messsystem zum Vermessen von Augenstrukturen aufweist, dessen Referenzarm in einem beweglichen Applikationskopf angeordnet ist.
- Figur 4:: zeigt schematisch im Querschnitt eine ophthalmologische Vorrichtung zur Behandlung von Augengewebe mittels Laserpulsen, welche ein interferometrisches Messsystem zum Vermessen von Augenstrukturen aufweist, dessen Referenzarm fest mit einem beweglichen Lichtprojektor verbunden ist.
- Figuren 5 und 6:: zeigen jeweils schematisch im Querschnitt eine ophthalmologische Vorrichtung zur Behandlung von Augengewebe mittels Laserpulsen, welche ein interferometrisches Messsystem zum Vermessen von Augenstrukturen aufweist, dessen Referenzarm eine veränderbare Länge hat und in einer Basisstation angeordnet ist.
- Figuren 7 und 8:: zeigen jeweils schematisch im Querschnitt eine ophthalmologische Vorrichtung zur Behandlung von Augengewebe mittels Laserpulsen, welche ein interferometrisches Messsystem zum Vermessen von Augenstrukturen mit einer flexiblen Lichtfaser im Messarm aufweist.
- Figur 9:: zeigt schematisch im Querschnitt eine ophthalmologische Vorrichtung zur Behandlung von Augengewebe mittels Laserpulsen, welche ein interferometrisches Messsystem mit Lichtquelle, Detektor, Messarm und Referenzarm zum Vermessen von Augenstrukturen aufweist.

### Wege zur Ausführung der Erfindung

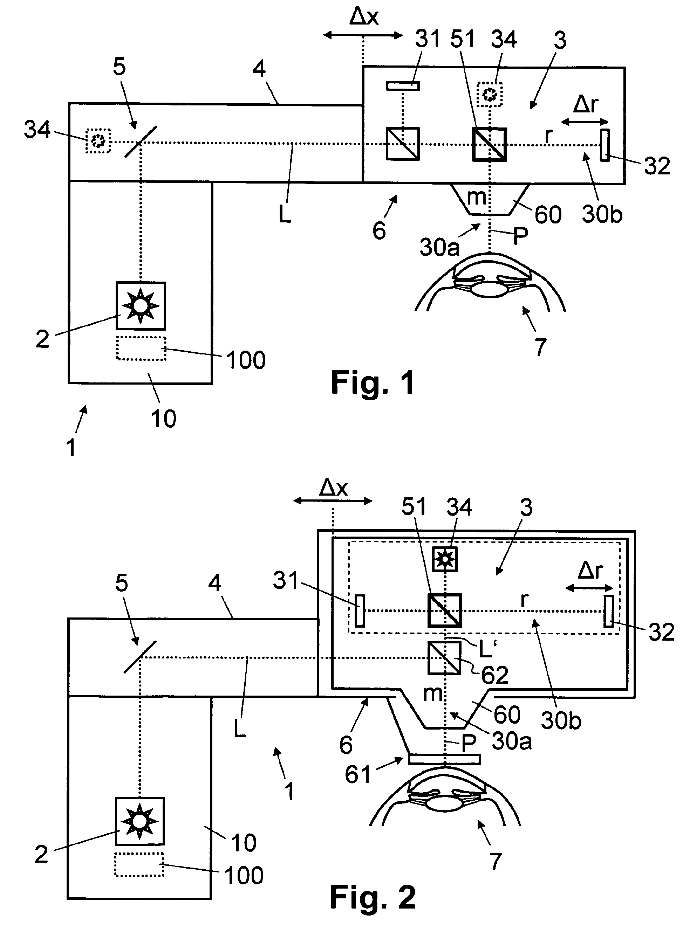

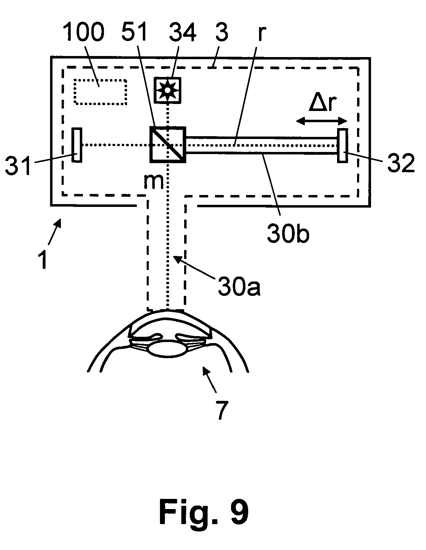

In den Figuren 1 bis 9 bezieht sich das Bezugszeichen 1 auf eine ophthalmologische Vorrichtung zur Behandlung von Augengewebe 7 mittels Laserpulsen P. Wie in den Figuren 1 bis 8 schematisch dargestellt ist, umfasst die ophthalmologische Vorrichtung 1 eine Lichtquelle 2 zur Erzeugung der Laserpulse P und einen Lichtprojektor 60 zur fokussierten Projektion der Laserpulse P ins Augengewebe 7. Die Lichtquelle 2 ist beispielsweise eine Laserquelle zur Erzeugung eines mit Femtosekundenlaserpulsen gepulsten Laserstrahls. Die Laserpulse P (Femtosekundenlaserpulse) werden über ein optisches Übertragungssystem 5 entlang eines Lichtübertragungspfads L von der Lichtquelle 2 zum Lichtprojektor 60 übertragen. Das optische Übertragungssystem 5 umfasst einen oder mehrere strahlablenkende (jeweils mit verkippbaren/drehbaren Spiegeln ausgestattete) Scanner, auf die hier jedoch nicht näher eingegangen wird.

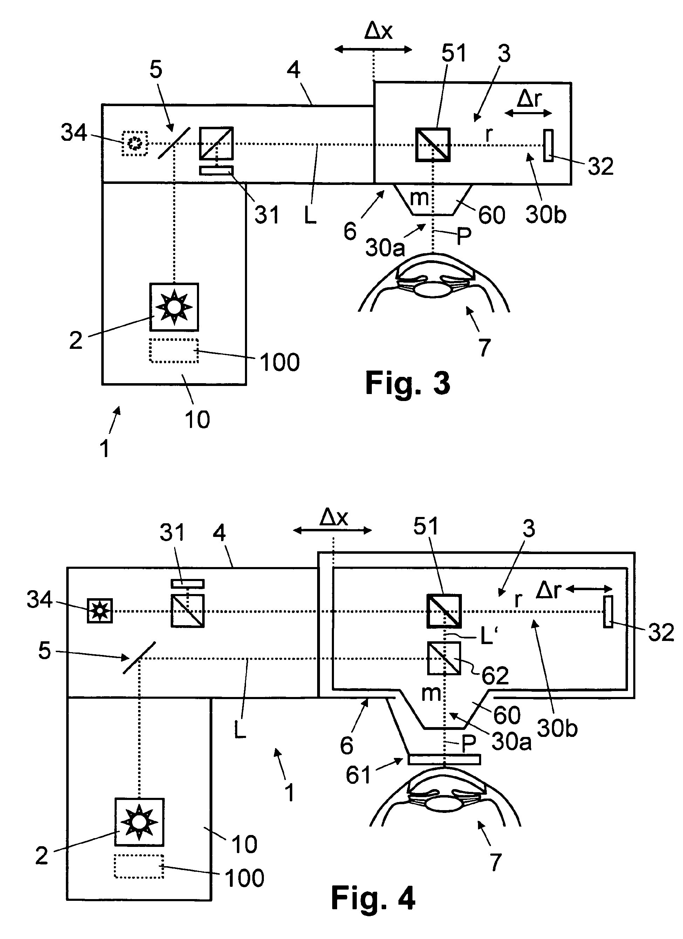

Wie in den Figuren 1 bis 8 illustriert, ist der Lichtprojektor 60 in einem Applikationskopf 6 angeordnet. Die ophthalmologische Vorrichtung 1 umfasst überdies eine Basisstation 10, an welcher der Applikationskopf 6 mit einem Arm 4 angebracht ist. Abhängig von der Ausführungsvariante ist der Arm 4 als flexibler Spiegelgelenkarm oder als in sich starrer Tragarm ausgeführt. In den Ausführungsvarianten, in denen der Arm 4 als flexibler Spiegelgelenkarm ausgeführt ist, ist der Applikationskopf 6 manuell auf das Auge aufsetzbar und über Befestigungsmittel 61 am Auge fixierbar, die in den Figuren 2 und 4 schematisch dargestellt sind. Die Befestigungsmittel 61 umfassen beispielsweise einen Saugring und sind mit einem transparenten Kontaktkörper versehen, der auf die Cornea eines zu behandelnden Auges aufgesetzt wird.

Der Lichtprojektor 60 ist relativ zur Lichtquelle 2 so bewegbar, dass die Länge des Lichtübertragungspfads L von der Lichtquelle 2 zum Lichtprojektor 60 veränderbar ist, wie in den Figuren 1 bis 8 mit dem Pfeil Δx angedeutet ist. Abhängig von der Ausführungsvariante ist der Lichtprojektor 60 im Applikationskopf 6 fest angebracht und wird zusammen mit dem Applikationskopf 6 relativ zur Lichtquelle 2 bewegt, wie beispielsweise in den Figuren 1, 3, und 5 bis 8 dargestellt ist, oder der Lichtprojektor 60 ist im Applikationskopf 6 beweglich ausgeführt und wird innerhalb des Applikationskopfs 6 relativ zum Applikationskopf 6 und relativ zur Lichtquelle 2 bewegt, wie beispielsweise in den Figuren 2 und 4 dargestellt ist.

Die ophthalmologische Vorrichtung 1 umfasst überdies ein interferometrisches Messsystem 3 zum Vermessen von Augenstrukturen (z.B. vordere und hintere Oberfläche der Cornea, vordere und hintere Oberfläche der Linse, die Retina, etc.) im Augengewebe 7. Das interferometrische Messsystem 3 ist für die optische Kohärenztomografie (OCT) mit Tiefenabtastung eingerichtet. Wie in den Figuren 1 bis 9 schematisch dargestellt ist, umfasst das interferometrische Messsystem 3 eine Lichtquelle 34, einen Messarm 30a, einen Referenzarm 30b, einen Strahlteiler 51 und einen Detektor 31. Der Strahlteiler 51 ist vorgesehen den von der Lichtquelle 34 erzeugten Lichtstrahl einerseits dem Messarm 30a und andererseits dem Referenzarm 30b zuzuführen. Der Strahlteiler 51 führt überdies die über den Messarm 30a empfangenen durch die Augenstrukturen im Augengewebe 7 reflektierten Lichtstrahlen und den über den Referenzarm 30b empfangenen vom Spiegel 32 des Referenzarms 30b reflektierten Lichtstrahl dem Detektor 31 zu. Der Detektor 31 ist eingerichtet, die Interferenzen zwischen dem reflektierten Messstrahl vom Messarm 30a und dem reflektierten Referenzstrahl vom Referenzarm 30a zur Bestimmung und Vermessung der Augenstrukturen im Augengewebe 7 zu detektieren. Wie die Figuren 1 bis 9 schematisch zeigen, weist der Messarm 30a vom Strahlteiler 51 zu den zu vermessenden Augenstrukturen des Augengewebes 7 eine einfache Weglänge m auf und der Referenzarm 30b hat vom Strahlteiler 51 zum Spiegel 32 eine einfache Weglänge r (gesamte Länge des Lichtübertragungspfad, inklusive Hin- und Rückweg, des Messarms 30a und des Referenzarms 30b beträgt 2m respektive 2r). Wie mit dem Pfeil Δr angedeutet ist, ist der Spiegel 32 des Referenzarms 30b in einer Ausführungsvariante bewegbar ausgeführt, so dass die Länge des Referenzarms 30b veränderbar ist.

Das interferometrische Messsystem 3 ist beispielsweise als Time Domain OCT (TOCT) mit veränderbarer Referenzarmlänge, breitbandiger Lichtquelle 34 und Detektor 31 mit einfacher aber hochempfindlicher Diode ausgeführt. Die Veränderung der Referenzarmlänge sowie unterschiedliche geeignete Methoden zur Signalverarbeitung werden z.B. in EP0581871 beschrieben.

In einer anderen Ausführungsvariante ist das interferometrische Messsystem 3 ohne veränderbare Referenzarmlänge für die statische Tiefenabtastung eingerichtet. In einer Ausführungsvariante ist das interferometrische Messsystem 3 als so genanntes Swept Source OCT (SSOCT) ausgeführt und umfasst eine Lichtquelle 34 mit veränderlicher Wellenlänge sowie einen Detektor 31 mit einer einfachen aber hochempfindlichen Photodiode.

Das interferometrische Messsystem 3 ist in einer anderen Variante als Spectral Domain OCT (SDOCT) mit breitbandiger Lichtquelle 34 und geeignetem Detektor 31 ausgeführt (z.B. Beugungsgitter mit CCD-Zeile).

In den nachfolgenden Abschnitten werden mit Bezug zu den Figuren 1 bis 8 verschiedene Varianten der ophthalmologischen Vorrichtung 1 mit unterschiedlichen Ausführungen des interferometrischen Messsystems 3 und unterschiedlichen Anordnungen der Komponenten des interferometrischen Messsystems 3 beschrieben. Dabei wird hier festgehalten, dass die oben angeführten Ausführungsvarianten, insbesondere die Bewegbarkeit des Lichtprojektors 60 mit oder relativ zum Applikationskopf 6, der bewegliche oder starr ausgeführte Arm 4 und die Ausführung mit oder ohne Befestigungsmittel 61, mit den nachfolgenden Ausführungsvarianten des interferometrischen Messsystems 3 kombinierbar sind, auch wenn nicht sämtliche Kombinationen explizit in einer der Figuren 1 bis 8 dargestellt sind.

In den Ausführungsvarianten der Figuren 1 bis 7 ist das interferometrische Messsystem 3 in den Lichtübertragungspfad L von der Lichtquelle 2 zum Lichtprojektor 60 eingekoppelt, welcher für die Behandlung des Augengewebes 7 mittels Laserpulsen vorgesehen ist. In diesen optisch eingekoppelten Ausführungsvarianten des interferometrischen Messsystems 3 ist in der ophthalmologischen Vorrichtung 1 jeweils ein bezüglich der Lichtquelle 2 bewegbares optisches Element vorgesehen, das verhindert, dass sich die Weglängendifferenz (m-r respektive 2(m-r)) von Messarm 30a und Referenzarm 30b aufgrund einer durch die Bewegung des Lichtprojektors 60 bewirkten Längenveränderung Δx des Lichtübertragungspfads L verändert.

In den Varianten der Figuren 1 bis 4 ist das bewegbare optische Element ein fest mit dem Lichtprojektor 60 verbundener Strahlteiler 51, über welchen der Messarm 30a und der Referenzarm 30b des interferometrischen Messsystems 3 gekoppelt sind. Wie in den Figuren 1 bis 4 ersichtlich ist, ist der Referenzarm 30b des interferometrischen Messsystems 3 in diesen Varianten im Applikationskopf 6 angeordnet und wird zusammen mit dem Lichtprojektor 60 und dem damit verbundenen Strahlteiler 51 bewegt, zusammen mit dem Applikationskopf 6 (Figuren 1 und 3) oder innerhalb des Applikationskopfs 6 relativ zum Applikationskopf 6 (Figuren 2 und 4). In den Varianten der Figuren 1 und 2 ist auch der Detektor 31 des interferometrischen Messsystems 3 im Applikationskopf 6 angeordnet und wird zusammen mit dem Applikationskopf 6 (Figur 1) oder innerhalb des Applikationskopfs 6 relativ zum Applikationskopf 6 (Figur 2) bewegt. In den Varianten der Figuren 3 und 4 ist der Detektor 31 des interferometrischen Messsystems 3 ausserhalb des Applikationskopfs 6 angeordnet, beispielsweise im Arm 4 oder in der Basisstation 10.

In verschiedenen Varianten ist das interferometrische Messsystem 3 über den Strahlteiler 51 direkt in den Lichtübertragungspfad L von der Lichtquelle 2 zum Lichtprojektor 60 eingekoppelt (z.B. in den Figuren 1, 3 und 5) oder das interferometrische Messsystem 3 ist über einen weiteren Strahlteiler 62 in den Lichtübertragungspfad L eingekoppelt, welcher über einen separaten Lichtübertragungspfad L' mit dem Strahlteiler 51 des interferometrischen Messsystems 3 verbunden ist (z.B. Figuren 2, 4 und 6).

In den In den Varianten der Figuren 1 bis 4 ist die Lichtquelle 34 des interferometrischen Messsystems 3 je nach Variante im Applikationskopf 6 (z.B. Figuren 1 oder 2) oder im Arm 4 oder in der Basisstation 10 (z.B. Figuren 1, 3 oder 4) angeordnet. In alternativen Ausführungsvarianten dient die Lichtquelle 2 auch als Lichtquelle für das interferometrische Messsystem 3 (z.B. Figuren 1 oder 3).

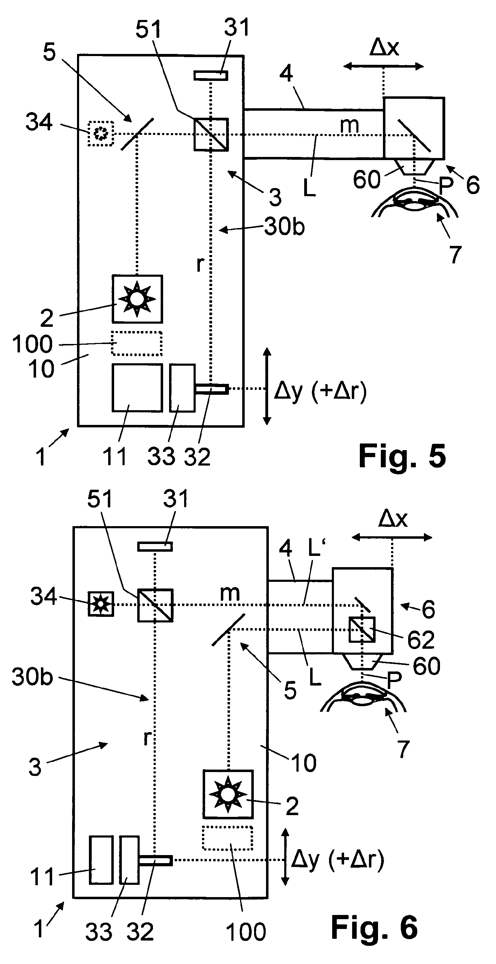

In den Ausführungsvarianten der Figuren 5 und 6 ist das bewegbare optische Element ein im Referenzarm 30b angeordneter Spiegel 32, der zur Kompensation einer Bewegung Δx des Lichtprojektors 60 so bewegt wird, wie mit dem Pfeil Δy angedeutet ist (Δy=kΔx, wobei k eine Systemkonstante ist), dass eine durch die Bewegung Δx des Lichtprojektors 60 bewirkte Veränderung die Weglängendifferenz (m-r) von Messarm 30a und Referenzarm 30b verhindert wird. Dazu umfasst die ophthalmologische Vorrichtung 1 ein Steuermodul 11 und einen mit dem Spiegel 32 des Referenzarms 30b gekoppelten Bewegungstreiber 33, welche eingerichtet sind, den Spiegel 32 so zu verschieben, dass die Weglänge r des Referenzarms 30 entsprechend der durch die Bewegung des Lichtprojektors 60 bewirkten Veränderung Δx der Länge des Lichtübertragungspfads L angepasst wird. Das Steuermodul 11 ist beispielsweise als programmiertes Softwaremodul zur Steuerung eines Prozessors der ophthalmologischen Vorrichtung 1 oder als hardwaremässig ausgeführtes Logikmodul ausgeführt.

Sämtliche Ausführungsvarianten können überdies mit zusätzlichen Strahlteilern, optisch abbildenden Elementen und Lichtleitfasern kombiniert werden. Insbesondere kann die Einkopplung des interferometrischen Messsystems 3 in den Lichtübertragungspfad L über optische Fasern erfolgen. Beispielsweise erfolgt die Einkopplung des interferometrischen Messsystems 3 in den Lichtübertragungspfad L in einer Untervariante der Ausführungsvariante gemäss Figur 5 über eine optische Faser und einen strahlteilenden Spiegel in einem als Gelenkarm ausgeführten Arm 4. In einem anderen Beispiel erfolgt die Einkopplung des interferometrischen Messsystems in den Lichtübertragungspfad L' in einer Untervariante der Ausführungsvariante gemäss Figur 6 ebenfalls über eine optische Faser.

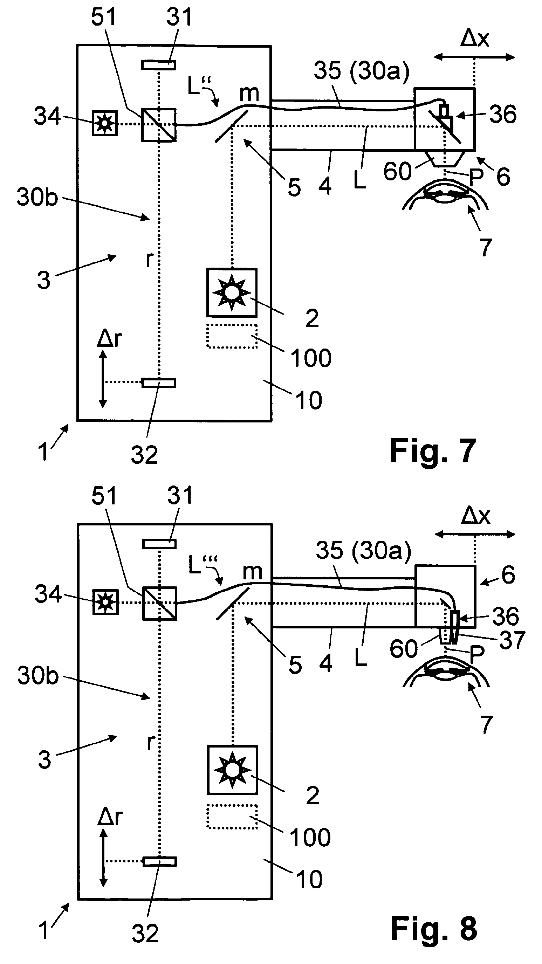

In der Variante der Figur 7 ist das bewegbare optische Element eine fest mit dem Lichtprojektor 60 verbundene Lichtleiterkopplung 36, welche den Lichtprojektor 60 über eine flexible Lichtfaser 35 in das interferometrische Messsystem 3 einbindet. Die flexible Lichtfaser 35 bildet einen vom Lichtübertragungspfad L von der Lichtquelle 2 zum Lichtprojektor 60 separaten Lichtübertragungspfad L", über den der Strahlteiler 51 des interferometrischen Messsystems 3 mit dem Lichtprojektor 60 verbunden ist. Die Länge der Lichtfaser 35 respektive des separaten Lichtübertragungspfads L" vom Strahlteiler 51 des interferometrischen Messsystems 3 zum Lichtprojektor 60 bleibt unabhängig von einer durch die Bewegung Δx des Lichtprojektors 60 bewirkten Längenänderung des Lichtübertragungspfad L von der Lichtquelle 2 zum Lichtprojektor 60 konstant. Somit bleibt die Weglängendifferenz (m-r) von Messarm 30a und Referenzarm 30b unverändert.

In der Variante gemäss der Figur 8 ist zusätzlich zu dem für die fokussierte Projektion der Laserpulse P ins Augengewebe 7 vorgesehenen Lichtprojektor 60 ein weiterer Lichtprojektor 37 vorgesehen, welcher zur Projektion des Messstrahls des interferometrischen Messsystems 3 im Messarm 30a dient. Dieser zusätzliche Lichtprojektor 37 ist fest mit dem Lichtprojektor 60 verbunden und wird folglich bei dessen Bewegungen mitbewegt. In der Variante der Figur 8 ist als weiteres bewegbares optisches Element eine fest mit den Lichtprojektoren 60 und 37 verbundene Lichtleiterkopplung 36 vorgesehen, welche den zusätzlichen Lichtprojektor 37 - wie obenstehend im Zusammenhang mit der Figur 7 beschrieben wurde - über die flexible Lichtfaser 35 in das interferometrische Messsystem 3 einbindet. Die flexible Lichtfaser 35 bildet einen vom Lichtübertragungspfad L von der Lichtquelle 2 zum Lichtprojektor 60 separaten Lichtübertragungspfad L"', über den der Strahlteiler 51 des interferometrischen Messsystems 3 mit dem zusätzlichen Lichtprojektor 37 verbunden ist, so dass auch hier die Weglängendifferenz (m-r) von Messarm 30a und Referenzarm 30b bei Bewegungen Δx des Lichtprojektors 60 bezüglich der Lichtquelle 2 unverändert bleibt.

Abschliessend soll festgehalten werden, dass die ophthalmologische Vorrichtung 1 in einer Variante mit einem zusätzlichen Korrekturmodul 100 versehen ist, das für die numerische Korrektur von Restfehlern eingerichtet ist, die in der Praxis auch beim Einsatz der oben beschriebenen Korrektur- respektive Kompensationsmassnahmen zur Verhinderung von Änderungen in der Weglängendifferenz (m-r) zwischen Messarm 30a und Referenzarm 30b bei Bewegungen Δx des Lichtprojektors 60 bezüglich der Lichtquelle 2 verbleiben können, beispielsweise aufgrund von Regelungsabweichungen oder bei stufenweisem Verfahren des Referenzarms 30b. Das Korrekturmodul 100 ist beispielsweise als programmiertes Softwaremodul zur Steuerung eines Prozessors der ophthalmologischen Vorrichtung 1 oder als hardwaremässig ausgeführtes Logikmodul ausgeführt. In einer Variante ist das Korrekturmodul 100 eingerichtet, die Korrektur respektive Kompensation von sich bei Bewegungen Δx des Lichtprojektors 60 bezüglich der Lichtquelle 2 ergebenden Änderungen in der Weglängendifferenz (m-r) zwischen Messarm 30a und Referenzarm 30b vollständig numerisch auszuführen, beispielsweise als Alternative zu den oben beschriebenen Korrektur- respektive Kompensationsmassnahmen oder als Backup-Lösung bei einem Ausfall des Steuermoduls 11 oder des Bewegungstreibers 33. Das Korrekturmodul 100 ist eingerichtet, die Korrektur von Änderungen in der Weglängendifferenz (m-r) auf der Grundlage der vom interferometrischen Messsystem 3 erfassten Mess- respektive Bilddaten der Augenstrukturen durchzuführen, beispielsweise durch die Detektierung und Beseitigung von Sprüngen und Verschiebungen im Konturverlauf der Augenstrukturen. Die erfassten Konturen der Augenstrukturen werden beispielsweise als Linien mittels Bildpunkten in einem Array abgebildet und Änderungen in der Weglängendifferenz (m-r) aufgrund von Sprüngen und Verschiebungen bestimmt, die beispielsweise mittels geeigneter Bildverarbeitungsalgorithmen über mehrere Linien hinweg - also über die Konturverläufe mehrerer, in unterschiedlicher Tiefe des Augengewebes angeordneten Augenstrukturen - detektiert werden.

## Patentansprüche

1. Ophthalmologische Vorrichtung (1) zur Behandlung von Augengewebe (7) mittels Laserpulsen (P), umfassend:
eine Basisstation (10),
eine in der Basisstation (10) angeordnete Lichtquelle (2) zur Erzeugung der Laserpulse (P),
einen Lichtprojektor (60) zur fokussierten Projektion der Laserpulse (P) ins Augengewebe (7), wobei der Lichtprojektor (60) bezüglich der genannten Lichtquelle (2) bewegbar ist, so dass die Länge eines Lichtübertragungspfads (L) von der Lichtquelle (2) zum Lichtprojektor (60) veränderbar ist,
ein interferometrisches Messsystem (3) zum Vermessen von Augenstrukturen, und
ein bezüglich der genannten Lichtquelle (2) bewegbares optisches Element (51, 32), das vorgesehen ist, eine Veränderung der Weglängendifferenz von Messarm (30a) und Referenzarm (30b) des interferometrischen Messsystems (3) aufgrund einer durch eine Bewegung des Lichtprojektors (60) bewirkten Veränderung (Δx) der Länge des Lichtübertragungspfads (L) zu verhindern,
**dadurch gekennzeichnet, dass** das bewegbare optische Element ein Spiegel (32) ist, der im in der Basisstation (10) angeordneten Referenzarm (30b) angeordnet ist, und
dass die ophthalmologische Vorrichtung (1) ein Steuermodul (11) und einen Bewegungstreiber (33) umfasst, die eingerichtet sind, den Spiegel (32) in der Basisstation (10) zur Kompensation der Bewegung (x) des Lichtprojektors (60) so zu verschieben, dass die Weglänge des Referenzarms (30) entsprechend einer durch eine Bewegung des Lichtprojektors (60) bewirkten Veränderung (Δx) der Länge des Lichtübertragungspfads (L) angepasst wird.

2. Ophthalmologische Vorrichtung (1) nach Anspruch 1, **dadurch gekennzeichnet, dass** das interferometrische Messsystem (3) in den genannten Lichtübertragungspfad (L) eingekoppelt ist.

3. Ophthalmologische Vorrichtung (1) nach einem der Ansprüche 1 oder 2, **dadurch gekennzeichnet, dass** die ophthalmologische Vorrichtung (1) einen auf das Auge applizierbaren Applikationskopf (6) umfasst, und dass der Lichtprojektor (60) im Applikationskopf (6) angeordnet ist.

4. Ophthalmologische Vorrichtung (1) nach einem der Ansprüche 1 bis 3, **dadurch gekennzeichnet, dass** das interferometrische Messsystem (3) einen Detektor (31) umfasst, welcher extern zum Applikationskopf (6) angeordnet ist.

5. Ophthalmologische Vorrichtung (1) nach einem der Ansprüche 1 bis 4, **dadurch gekennzeichnet, dass** der Lichtprojektor (60) über einen Arm (4) mit der Basisstation (10) verbunden ist.

6. Ophthalmologische Vorrichtung (1) nach einem der Ansprüche 1 bis 5, **dadurch gekennzeichnet, dass** das interferometrische Messsystem (3) eine Lichtquelle (34) umfasst, welche in der Basisstation (10) angeordnet ist, und dass das interferometrische Messsystem (3) einen Detektor (31) umfasst, welcher in der Basisstation (10) angeordnet ist.

7. Ophthalmologische Vorrichtung (1) nach einem der Ansprüche 1 bis 6, **dadurch gekennzeichnet, dass** das interferometrische Messsystem (3) einen Referenzarm (30) mit veränderbarer Referenzarmlänge (r) aufweist.

## Claims

1. Ophthalmic device (1) for treating eye tissue (7) by means of laser pulses (P), comprising:
a base station (10,
a light source (2) arranged in the base station (10) for generating the laser pulses (P),
a light projector (60) for focused projection of the laser pulses (P) into the eye tissue (7), wherein the light projector (60) is movable in relation to the aforementioned light source (2) in such a way that the length of a light-transmission path (L) from the light source (2) to the light projector (60) is changeable,
an interferometric measurement system (3) for measuring eye structures, and
an optical element (51, 32), which is movable in relation to the aforementioned light source (2) and provided for preventing a change in the path length difference between measurement arm (30a) and reference arm (30b) of the interferometric measurement system (3) as a result of a change (Δx) in the length of the light-transmission path (L) caused by a movement of the light projector (60),
**characterized in that** the movable optical element is a mirror (32) which is arranged in the reference arm (30b) arranged in the base station (10) and
**in that** the ophthalmic device (1) comprises a control module (11) and a movement driver (33), which are configured to displace the mirror (32) in the base station (10) in such a way to compensate the movement (x) of the light projector (60) that the path length of the reference arm (30) is adapted in accordance with a change (Δx) in the length of the light-transmission path (L) caused by a movement of the light projector (60).

2. Ophthalmic device (1) according to Claim 1, **characterized in that** the interferometric measurement system (3) is coupled into the aforementioned light-transmission path (L).

3. Ophthalmic device (1) according to either of Claims 1 or 2, **characterized in that** the ophthalmic device (1) comprises an application head (6) which can be applied onto the eye and **in that** the light projector (60) is arranged in the application head (6).

4. Ophthalmic device (1) according to one of Claims 1 to 3, **characterized in that** the interferometric measurement system (3) comprises a detector (31), which is arranged outside of the application head (6).

5. Ophthalmic device (1) according to one of Claims 1 to 4, **characterized in that** the light projector (60) is connected to the base station (10) via an arm (4).

6. Ophthalmic device (1) according to one of Claims 1 to 5, **characterized in that** the interferometric measurement system (3) comprises a light source (34), which is arranged in the base station (10), and **in that** the interferometric measurement system (3) comprises a detector (31), which is arranged in the base station (10).

7. Ophthalmic device (1) according to one of Claims 1 to 6, **characterized in that** the interferometric measurement system (3) has a reference arm (30) with a changeable reference arm length (r).

## Revendications

1. Dispositif ophtalmologique (1) destiné au traitement de tissu oculaire (7) au moyen d'impulsions laser (P), comprenant:
une station de base (10),
une source de lumière (2) disposée dans la station de base (10) et destinée à générer l'impulsion laser (P), un projecteur de lumière (60) destiné à la projection concentrée de l'impulsion laser (P) dans le tissu oculaire (7), le projecteur de lumière (60) pouvant être déplacé par rapport à ladite source de lumière (2), de sorte que la longueur d'un trajet de transmission de lumière (L) de la source de lumière (2) au projecteur de lumière (60) puisse être modifiée,
un système de mesure interférométrique (3) destiné à mesurer les structures de l'oeil, et
un élément optique (51, 32) mobile par rapport à ladite source de lumière (2), lequel est conçu pour empêcher une modification de la différence de longueur de trajet entre le bras de mesure (30a) et le bras de référence (30b) du système de mesure interférométrique (3) en raison d'une modification (Δx) de la longueur du trajet de transmission de lumière (L) provoquée par un mouvement du projecteur de lumière (60),
**caractérisé en ce que** l'élément optique mobile est un miroir (32) qui est monté dans le bras de référence (30b) disposé dans la station de base (10), et
**en ce que** le dispositif ophtalmologique (1) comprend un module de commande (11) et un mécanisme de déplacement (33) qui sont conçus pour décaler le miroir (32) dans la station de base (10) en vue de compenser le mouvement (x) du projecteur de lumière (60), de telle sorte que la longueur de course du bras de référence (30) est adaptée en fonction d'une modification (Δx) de la longueur du trajet de transmission de lumière (L) provoquée par un mouvement du projecteur de lumière (60).

2. Dispositif ophtalmologique (1) selon la revendication 1, **caractérisé en ce que** le système de mesure interférométrique (3) est incorporé par couplage dans ledit trajet de transmission de lumière (L).

3. Dispositif ophtalmologique (1) selon l'une des revendications 1 ou 2, **caractérisé en ce que** le dispositif ophtalmologique (1) comprend une tête d'application (6) pouvant être appliquée sur l'oeil, et **en ce que** le projecteur de lumière (60) est disposé dans la tête d'application (6).

4. Dispositif ophtalmologique (1) selon l'une des revendications 1 à 3, **caractérisé en ce que** le système de mesure interférométrique (3) comprend un détecteur (31) qui est disposé à l'extérieur de la tête d'application (6).

5. Dispositif ophtalmologique (1) selon l'une des revendications 1 à 4, **caractérisé en ce que** le projecteur de lumière (60) est relié à la station de base (10) par le biais d'un bras (4).

6. Dispositif ophtalmologique (1) selon l'une des revendications 1 à 5, **caractérisé en ce que** le système de mesure interférométrique (3) comprend une source de lumière (34) qui est disposée dans la station de base (10), et **en ce que** le système de mesure interférométrique (3) comprend un détecteur (31) qui est disposé dans la station de base (10).

7. Dispositif ophtalmologique (1) selon l'une des revendications 1 à 6, **caractérisé en ce que** le système de mesure interférométrique (3) possède un bras de référence (30) ayant une longueur de bras de référence (r) variable.
